# DEMANDE DE BREVET EUROPEEN

(11) **EP 0 955 096 A1**
(43) Date de publication de la demande: **10.11.1999**
(21) Numéro de dépôt: 99400737.5
(22) Date de dépôt: 25.03.1999
(51) Int. Cl.: B01J 37/20, C10G 45/08, C07C 7/163

(54) **Procédé de sulfuration d'un catalyseur au moyen d'un polysulfure organique et procédé de purification de naphtalène utilisant le catalyseur sulfuré**

(30) Priorité: 06.05.1998 FR 9805740
(71) Demandeur: INSTITUT FRANCAIS DU PETROLE, 92852 Rueil-Malmaison Cedex (FR)
(72) Inventeur: Mignard, Samuel, 78400 Chatou (FR); Guitton, Corinne, 78400 Chatou (FR); Kasztelan, Slavik, 92500 Rueil Malmaison (FR)
(74) Mandataire: Andréeff, François

(57) **Abrégé**

Un procédé de sulfuration d'un catalyseur contenant de l'alumine et au moins un métal du groupe VIII à l'état d'oxydes, ce procédé consiste à injecter sur le catalyseur au moins un polysulfure organique choisi dans le groupe formé par les polysulfures organiques de type TPS 32®, TPS 37®, TPS 40®, TPS 54®, de façon telle que la quantité, en masse, de polysulfure organique injectée avant que la température du catalyseur atteigne 250°C. par rapport à la quantité, en masse, de soufre nécessaire à la sulfuration complète dudit catalyseur soit au moins égale à 1.56 pour les polysulfures de type TPS 32®, au moins égale à 1.35 pour les polysulfures de type TPS 37®, au moins égale à 1,25 pour les polysulfures de type TPS 40®, et au moins égale à 0,93 pour les polysulfures de type TPS 54®.

## Description

L'invention concerne un procédé de sulfuration d'un catalyseur au moyen d'un composé polysulfure organique, le catalyseur obtenu par ce procédé, ainsi qu'un procédé de purification d'une coupe naphtalènique réalisé avec un catalyseur sulfuré par un composé polysulfure organique.

Parmi les produits issus de la distillation des goudrons de houille se trouve une fraction naphtalénique contenant essentiellement du naphtalène, cette fraction comprend également, de façon non limitative, des produits soufrés tel que par exemple le benzothiophène, des produits azotés telle que par exemple la quinoléine, des produits oxygénés tels que par exemple des dérivés phénoliques et des hydrocarbures insaturés tel que l'indène.

Actuellement, il existe deux types de naphtalènes sur le marché. Le premier type est un "naphtalène technique" dont la pureté est supérieure à 98%. Le deuxième type est un "naphtalène pur" principalement utilisé pour la synthèse et la fabrication de produits anti-insectes (la "naphtaline"). Ce produit doit être parfaitement incolore, il doit donc être très pur. Sa pureté doit être supérieure à 99,96 % et la teneur en soufre pratiquement nulle (quelques ppm poids).

La coupe naphtalènique traitée par le procédé de purification selon l'invention peut contenir plus de 50% en poids de naphtalène, avantageusement plus de 75% en poids, et le plus souvent plus de 85% en poids. Elle contient également des composés soufrés représentant, par rapport à la charge totale, jusqu'à 5% en poids de soufre mais le plus souvent moins de 1% en poids, des composés azotés sous forme par exemple de quinoléine (jusqu'à 1% en poids), des monooléfines comme, par exemple de l'indène (jusqu'à 1% en poids mais le plus souvent moins de 0,5% en poids) et des composés oxygénés comme par exemple les phénols (jusqu'à 1% en poids mais le plus souvent moins de 0,5% en poids).

Des procédés de purification de coupes naphtalène existent déjà.

Ainsi, la demande de brevet JP-05-017,376 décrit un procédé d'hydrogénation du naphtalène à basse pression (0-20 bars) dans lequel les impuretés sont hydrogénées ainsi qu'une partie du naphtalène et une partie de la tétraline formée.

Une autre demande JP-05-085,960 décrit un procédé de purification d'une coupe naphtalènique comprenant une première étape d'hydrogénation en phase liquide sous faible ou moyenne pression (0-20 bars), à 100-300°C, avec un catalyseur choisi dans le groupe formé par les catalyseurs Ni-Co-Mo, platine sur charbon, Pt-Ni-Mo, Pd-alumine, CoMo-alumine. L'effluent obtenu est, dans une seconde étape, dégazé pour éliminer H₂S, NH₃ et l'éthylbenzène. Puis, l'effluent est lavé avec un acide inorganique (troisième étape) et ensuite séparé (quatrième étape). L'effluent ainsi obtenu est déshydraté par distillation azéotropique avec l'éthylbenzène (cinquième étape). Les impuretés résiduelles sont adsorbées (sixième étape) par de l'argile. L'effluent peut alors être distillé (septième étape) et pressé (huitième étape) pour obtenir le naphtalène purifié.

La présente invention concerne un procédé de sulfuration d'un catalyseur au moyen d'un composé polysulfure organique, le catalyseur obtenu par ce procédé, ainsi qu'un procédé de purification des coupes naphtalèniques effectué en présence d'un catalyseur sulfuré par au moins un polysulfure organique.

Le procédé de sulfuration d'un catalyseur contenant au moins de l'alumine et au moins un métal du groupe VIII à l'état d'oxydes selon la présente invention consiste à injecter sur le catalyseur au moins un polysulfure organique choisi dans le groupe formé par les polysulfures organiques de type TPS 32®, TPS 37®, TPS 40®, TPS 54®, de façon telle que la quantité, en masse, de polysulfure organique injectée avant que la température du catalyseur atteigne 250°C, par rapport à la quantité, en masse, de soufre nécessaire à la sulfuration complète dudit catalyseur soit au moins égale à 1,56 pour les polysulfures organiques de type TPS 32®, au moins égale à 1,35 pour les polysulfures organiques de type TPS 37®, au moins égale à 1,25 pour les polysulfures organiques de type TPS 40®, au moins égale à 0,93 pour les polysulfures organiques de type TPS 54®.

Les polysulfures organiques appelés TPS sont, par exemple, décrits dans le brevet français FR 2548205.

Au sens de la présente description "QA" représente la quantité, en masse, de polysulfure organique injectée et représente "QS₀" la quantité, en masse, de soufre nécessaire à la sulfuration complète du métal (ou des métaux) du catalyseur.

Le procédé de purification des coupes naphtalènes de la présente invention utilise un catalyseur traité selon le procédé de sulfuration selon la présente invention.

Ce catalyseur permet d'hydrotraiter la charge de façon sélective de façon à pouvoir, uniquement par cristallisation, séparer le naphtalène purifié. Cet hydrotraitement permet d'éliminer en une seule étape la plus grande partie des impuretés soufrées, azotées, oxygénées, oléfiniques tout en limitant l'hydrogénation Une hydrogénation limitée permet la réduction de la quantité de tétraline et de décaline dans le produit final.

Le procédé de purification selon la présente invention comprend généralement les étapes suivantes : une première étape qui a pour but d'abaisser la teneur en soufre présent sous forme de molécules soufrées à la valeur désirée, puis successivement au moins une étape dans laquelle une partie au moins de l'effluent qui a subi l'hydrotraitement est débarrassé au moins en partie sinon en totalité des molécules de H₂S, NH₃ et H₂O générées, au moins une étape pour récupérer la plus grande quantité de naphtalène avec la pureté la plus élevée possible.

La première étape qui a pour but d'abaisser la teneur en soufre présent sous forme de molécules soufrées à la valeur désirée, les molécules soufrées sont alors transformées en molécules désulfurées et H₂S. Dans cette première étape, les molécules azotées sont largement transformées en molécules déazotées et NH₃. Cette étape permet aussi d'hydrogéner les molécules oléfiniques et de "déshydroxyler" les molécules contenant un groupement OH.

Au cours de cette étape, le catalyseur et les conditions opératoires sont choisis de façon à réaliser un hydrotraitement poussé tout en limitant l'hydrogénation du naphtalène en tétraline et décaline. En effet, la tétraline et la décaline peuvent être ici considérées comme des sous-produits de la réaction et sont alors indésirables. Au cours de ladite étape, il ne se forme pratiquement pas de tétraline ni de décaline (généralement moins de 5% en poids dans les effluents et de préférence moins de 3% en poids).

Par hydrotraitement poussé, on comprend une désulfuration de la charge initiale d'au moins 70% et de préférence d'au moins 90% et de façon encore plus préférée d'au moins 98%, une déazotation d'au moins 50% et de façon préférée d'au moins 80%, une hydrogénation des oléfines d'au moins 80% et de préférence d'au moins 95% et une déshydroxylation d'au moins 75% et de préférence d'au moins 90%.

Les conditions opératoires de cette étape sont les suivantes. La pression totale est comprise entre 0,1 MPa (1 bar) et 0,9 MPa et de façon préférée entre 0,2 MPa et 0,9 MPa et de façon encore plus préférée entre 0,2 MPa et 0,8 MPa. La température de réaction est comprise entre 150°C et 325°C et de préférence entre 200°C et 320°C et de façon encore plus préférée entre 220°C et 300°C. La vitesse volumique horaire (VVH) est comprise entre 0,05 h⁻¹ et 10 h⁻¹ et de préférence entre 0,1 h⁻¹ et 5 h⁻¹ et de façon encore plus préférée entre 0,15 h⁻¹ et 2 h⁻¹. Le rapport molaire hydrogène/naphtalène à l'entrée du réacteur est compris entre 0,1 et 1,3 et de façon préférée inférieure à 1.

On mettra ensuite en oeuvre une étape dans laquelle une partie au moins de l'effluent qui a subi l'hydrotraitement sera débarrassé au moins en partie sinon en totalité des molécules de H₂S, NH₃ et H₂O générées. Ainsi, au moins 95% et mieux au moins 99% des molécules de H₂S, NH₃ et H₂O générées seront éliminées. Toute méthode permettant de réaliser ces séparations est utilisable. De façon usuelle, une simple colonne de strippage sera utilisée.

La troisième étape a pour but de récupérer la plus grande quantité de naphtalène avec la pureté la plus élevée possible. Toute méthode de séparation peut être utilisée, comme par exemple une distillation. La pureté du naphtalène est alors de l'ordre de 99,6%. Pour obtenir une pureté supérieure (par exemple, supérieure à 99,96%), il est particulièrement avantageux d'utiliser un procédé de cristallisation tel que le procédé de purification par cristallisation en milieu fondu PROABD® commercialisé par la société BEFS-PROKEM ou tout autre procédé de purification par cristallisation en milieu fondu. Cette étape aura essentiellement pour rôle de séparer le naphtalène de la tétraline et de la décaline obtenues par hydrogénation.

Le procédé de cristallisation PROABD® permet la séparation du mélange obtenu après hydrogénation. Ce procédé présente une haute efficacité sur les impuretés du naphtalène pur et permet donc d'atteindre une haute pureté.

L'étape de séparation peut mettre en oeuvre des techniques de lavage des cristaux de naphtalène pur soit par la technique de fusion fractionnée soit par la technique du lavage à l'aide d'une partie du produit purifié qui est alors alimenté à contre-courant du procédé de purification.

L'effluent strippé est soumis à un traitement par cristallisation se déroulant de la façon suivante : les opérations de cristallisation s'effectuent dans un appareil entièrement automatisé sans aucune intervention manuelle. Un fluide circule dans l'appareil. Selon les étapes du procédé de séparation, ce fluide est alternativement chauffé ou refroidi par l'intermédiaire d'échangeurs de chaleur. Une régulation fine permet d'apporter avec exactitude au naphtalène les calories ou frigories nécessaires à l'opération. Le naphtalène brut, préalablement fondu pour être introduit dans l'un des cristalliseurs, est soumis à une loi de descente en température sous inertage, ce qui provoque une cristallisation très controlée du produit. Au fur et à mesure du refroidissement, la quantité de cristaux augmente jusqu'à un certain taux prédéterminé ; solide et liquide sont alors séparés par égouttage. Pour parfaire la purification des cristaux, on procède :
- soit à une fusion partielle des cristaux (ressuage). En fondant, les cristaux génèrent un naphtalène pur qui se mélange au film résiduel de liquide et le purifie.
- soit à un lavage consistant en des séquences de remplissage/vidange de l'appareil par des liquides de plus en plus purs.

On passe ensuite à une ultime phase de l'étape de séparation : la fusion totale des cristaux purs résiduels. Suivant l'efficacité du procédé de cristallisation choisi et la pureté finale désirée, un ou plusieurs étages de cristallisation peuvent être utilisés.

De façon usuelle, le rendement en (tétraline + décaline) est inférieur à 10% poids et de façon préférée inférieure à 5% poids ou mieux inférieure ou égale à 3% poids (le rendement étant la masse de (tétraline + décaline) formée par rapport à la masse de naphtalène à la sortie du réacteur d'hydrotraitement).

De façon à augmenter le rendement en naphtalène, il est également possible de réaliser le recyclage de la tétraline et de la décaline c'est-à-dire d'ajouter tout ou partie de la tétraline et de la décaline récupérées après l'étape de séparation à la charge de départ. La quantité de (tétraline + décaline) introduite est dans le rapport pondéral (tétraline + décaline) /naphtalène compris entre 0,005 et 0,08.

Le catalyseur utilisé pour la première étape d'hydrotraitement du procédé de purification selon la présente invention est un catalyseur contenant au moins une matrice comprenant avantageusement de l'alumine et au moins un métal ayant une fonction hydro-déshydrogénante. De préférence, cette matrice ne contient pas de zéolithe. Ladite matrice peut également contenir au moins un élément choisi dans le groupe constitué par la silice-alumine, l'oxyde de bore, la magnésie, la zircone, les argiles et leurs mélanges.

La fonction hydro-déshydrogénante est assurée par au moins un métal ou composé de métal du groupe VIII auquel on ajoute éventuellement au moins un métal du groupe VIB de la classification périodique des éléments (Handbook of Chemistry and Physics, 76 th Edition, 1995-1996).

Dans la présente description les expressions « métal du groupe » et « élément du groupe » sont employées de façon indifférente.

Les éléments du groupe VIII sont, de préférence, le nickel et le cobalt et les éléments du groupe VIB sont, de préférence le molybdène et le tungstène.

La concentration totale en oxydes de métaux du groupe VIII et éventuellement du groupe VIB est comprise entre 5 et 40 % en poids et de préférence entre 7 et 30 % en poids. Lorsque le catalyseur contient au moins un métal ou composé de métal du groupe VIII et au moins un métal ou composé de métal du groupe VIB, le rapport pondéral (exprimé en oxyde métallique de métal) (ou métaux) du groupe VIB sur oxyde de métal (ou métaux) du groupe VIII est compris entre 1,25 et 20 et de préférence entre 2 et 10. De plus, ce catalyseur peut de préférence contenir du phosphore. La teneur en phosphore, exprimée en concentration en oxyde de phosphore P₂O₅, sera alors inférieure à 15 % en poids et de préférence inférieure à 10 % en poids par rapport au catalyseur fini.

De façon préférée, le catalyseur utilisé est de type CoMo déposé sur alumine.

Le catalyseur présente des caractéristiques très particulières :
- la surface spécifique BET est d'au plus 220m²/g, lorsque le catalyseur contient du phosphore, la surface spécifique BET est inférieure à 200 m²/g, de façon préférée la surface est d'au plus 180 m²/g.
- le volume poreux est compris entre 0,35 et 0,7 ml/g
- le diamètre moyen des pores est d'au moins 100Å (10⁻¹⁰ m) et de préférence compris entre 100 et 200Å, ce qui signifie que la fraction du volume poreux correspondant aux pores de diamètre inférieur à 90Å est faible, elle est généralement comprise entre 0 et 15% par rapport au volume poreux total.

Avant de pouvoir être utilisé à l'étape d'hydrotraitement d'un procédé de purification selon la présente invention. le catalyseur doit subir une étape de sulfuration qui peut être réalisée dans l'unité elle-même.

On a maintenant découvert que, d'une manière surprenante, l'activité et la stabilité des catalyseurs d'hydrotraitement comprenant au moins un métal du groupe VIII et éventuellement au moins un métal du groupe VIB et notamment ceux de type NiMo, CoMo, NiW et CoW dépendent étroitement de la procédure utilisée pour effectuer cette dite sulfuration.

Ainsi, on a constaté que l'activité et la stabilité du catalyseur d'hydrotraitement utilisé dans le cadre du procédé de purification du naphtalène étaient particulièrement améliorées par une sulfuration suivant un procédé conforme à la présente invention.

L'étape d'hydrotraitement du procédé de purification selon la présente invention comprend l'injection d'une quantité bien déterminée du composé polysulfure organique à une température inférieure à 250°C environ de façon à assurer l'amorçage de la réaction, ce composé polysulfure organique est choisi dans le groupe formé par les composés organique de type TPS32®, TPS37®, TPS40® et TPS54®.

Bien entendu, ce qui importe, c'est la température du catalyseur au moment où il est soumis au passage du composé polysulfure organique de type TPS et non pas une température qui serait mesurée à un moment quelconque, à un endroit quelconque de l'unité.

Ainsi, le catalyseur traité selon l'invention, présente de meilleures performances c'est-à-dire une meilleure activité et/ou une meilleure stabilité.

Par "activité" au sens de la présente description, il faut entendre la performance du catalyseur durant les premières heures de son fonctionnement en présence de la charge à traiter dans les conditions de fonctionnement l'unité.

Par "stabilité" au sens de la présente description, on entend l'évolution de l'activité du catalyseur au cours du temps.

Par "charge à traiter" au sens de la présente description, on désigne la fraction d'hydrocarbures qui est normalement envoyée dans le réacteur catalytique pour y être transformée.

Lors de la sulfuration, le catalyseur dans lequel les métaux sont à l'état d'oxydes, se sulfure. La quantité de soufre fixée varie d'un catalyseur à un autre notamment suivant sa procédure de fabrication et la teneur en oxyde(s) déposé(s) sur le support. Il est possible, à partir de la composition du catalyseur, de calculer la quantité de soufre qui, au maximum, sera fixée par le catalyseur. La quantité de soufre stoechiométrique, notée QS₀, sera donc, par définition, la quantité de soufre nécessaire à la sulfuration complète du métal ou des mélanges de métaux utilisés à savoir, par exemple, du molybdène sous forme de disulfure de molybdène MoS₂, du cobalt sous forme d'octosulfure de nonacobalt Co₉S₈, du nickel sous forme de sulfure de nickel NiS et du tungstène sous forme de disulfure de tungstène WS₂.

Dans la suite du texte, nous utiliserons la notation QA/QS₀ qui représente la quantité, en masse, du composé polysulfure organique de type TPS introduite par rapport à la quantité, en masse, de soufre qui serait nécessaire à la sulfuration complète du catalyseur telle qu'elle est définie précédemment. Par exemple, si QA/QS₀=1, cela signifie que la masse du composé polysulfure organique de type TPS injectée est égale à la masse de soufre nécessaire à la sulfuration théorique complète du catalyseur.

De façon surprenante, nous avons découvert que, lors de la sulfuration, le rapport QA/QS₀ atteint avant que la température du catalyseur soit de 250°C, avait une très grande importance. En effet, si une valeur minimale de QA/QS₀ n'est pas atteinte, la sulfuration du catalyseur risque de ne pas être satisfaisante et, par là même, les performances catalytiques exprimées en terme d'activité et de stabilité risquent d'être détériorées.

La présente invention décrit un procédé de sulfuration particulier où la quantité de soufre injecté avant que la température du catalyseur atteigne 250°C est déterminée de façon très précise. Dans notre invention, la quantité, en masse, de polysulfure organique injectée avant que la température du catalyseur atteigne 250°C, par rapport à la quantité, en masse, de soufre nécessaire à la sulfuration complète dudit catalyseur doit au moins être égale à 1,56 pour les polysulfures organiques de type TPS 32®, au moins égale à 1,35 pour les polysulfures organiques de type TPS 37®, au moins égale à 1,25 pour les polysulfures organiques de type TPS 40®, et au moins égale à 0,93 pour les polysulfures organiques de type TPS 54®.

Le soufre dont il est question est un soufre particulier. En effet, il s'agit du soufre qui peut provoquer la sulfuration de la phase oxyde. Par contre, le soufre contenu dans le gas-oil lui-même ne peut pas réaliser la sulfuration de la phase oxyde à basse température car les molécules soufrées sont trop stables.

Selon l'invention, il conviendra d'abord d'utiliser unc charge de sulfuration très spécifique puisqu'elle devra contenir le composé polysulfure organique de type TPS 32®, TPS 37®, TPS 40®, TPS 54®. Actuellement, la plupart des sulfurations sont réalisées en phase liquide c'est-à-dire que le catalyseur, lorsque la réaction de sulfuration a lieu, est immergé dans une phase liquide. Cette phase liquide contient généralement un hydrocarbure comme, par exemple. un white spirit ou un gas-oil. Un des principaux avantages d'utiliser une phase liquide est que l'élévation de température due à l'exothermicité de la réaction de sulfuration, reste très faible car les calories engendrées sont aisément dispersées dans la phase liquide.

Le débit de la charge de sulfuration introduite dans le réacteur est calculé par rapport au volume de catalyseur chargé dans le réacteur où a lieu la réaction de sulfuration. Dans le cas des liquides, le débit de liquide, exprimé en volume par heure, est compris usuellement entre 0,10 et 50 fois le volume de catalyseur, de préférence entre 0,25 et 10 et de façon encore plus préférée entre 0,25 et 2.

La sulfuration peut cependant aussi être réalisée en phase gazeuse. Dans ce cas, le débit de gaz est exprimé en volume par heure est beaucoup plus élevé. La valeur la plus courante est comprise entre 1 et 10000 litres de gaz par litre de catalyseur, mesurés dans les conditions normales de température et de pression. et de façon préférée entre 10 et 5000 litres de gaz par litre de catalyseur.

L'augmentation de la température entre la température de départ et 250°C environ est effectuée par les moyens connus de l'homme de l'art à un rythme compatible avec la bonne tenue mécanique de l'unité et l'élimination des contraintes engendrées par la dilatation des différentes parties de cette unité. A titre indicatif, la montée en température est généralement de l'ordre de 5 à 25°C par heure mais elle peut aussi être réalisée par paliers comme indiqué dans les exemples suivants.

D'une façon générale, ce procédé de sulfuration du catalyseur comprend aussi un traitement à l'hydrogène du catalyseur, effectuée avant. pendant ou après la sulfuration. Généralement, le catalyseur est tout d'abord soumis à une atmosphère d'hydrogène à température relativement basse c'est à dire à une température qui est comprise entre la température ambiante (environ 20°C) et une température inférieure à 250°C environ et de façon usuelle inférieure à 200°C voire inférieure à 160°C. La valeur la plus courante de débit d'hydrogène est comprise entre 1 et 10000 litres de gaz par litre de catalyseur, mesurés dans les conditions normales de température et de pression. et de façon préférée entre 10 et 5000 litres de gaz par litre de catalyseur. Ensuite, la charge liquide peut être injectée. Rappelons que cette charge liquide peut être une charge particulière ou la charge à traiter elle-même à laquelle on ajoute un composé polysulfure organique de type TPS 32®, TPS 37®, TPS 40®, TPS 54®.

La sulfuration des catalyseurs suivant le procédé de l'invention se traduit par une amélioration des performances du catalyseur notamment pour les réactions d'hydrodésulfuration, hydrodéazotation, hydrodémétallation, hydrogénation des composés aromatiques et oléfiniques. Par amélioration des performances, il faut entendre amélioration de l'activité et/ou de la stabilité du catalyseur.

Les travaux de recherche effectués par la demanderesse l'ont conduit à découvrir que, d'une façon surprenante, l'utilisation de l'enchaînement hydrotraitement sélectif et poussé puis séparation permettait d'obtenir un naphtalène de haute pureté avec un rendement très élevé.

Les exemples suivants illustrent l'invention sans toutefois en limiter la portée.

Ils sont conduits sur une charge de naphtalène de type carbochimique dont l'analyse est donnée dans le tableau 1.

**Tableau I**

| | Teneur (%poids) |
|---|---|
| Benzothiophène soit soufre | 2,45 soit 0,586 |
| Naphtalène | 96,5 |
| Tétraline | 0 |
| Phénols | 0,3 |
| Indène | 0,15 |
| 1 et 2 méthyl-naphtalène | 0,2 |
| Quinoleine | 0,3 |

La purification de la charge a été réalisée par un hydrotraitement sélectif poussé suivi d'un strippage puis d'une distillation et enfin d'une cristallisation.

Les conditions opératoires de l'étape d'hydrotraitement du procédé de purification sont les suivantes : une température de 318°C, une pression totale de 0,5 MPa (5 bars), débit d'hydrogène : 125 l/l de charge soit un rapport molaire de 0,62 et VVH : 1 h⁻¹.

Le catalyseur utilisé contient 3 % en poids de CoO et 14 % en poids de MoO₃ déposé sur alumine. La surface spécifique du catalyseur est de 210 m²/g, le diamètre moyen des pores est égal à 110Å (110 10⁻¹⁰ m) et le volume poreux égal à 0,58 ml/g.

La totalité de l'effluent est soumis à un strippage de façon à éliminer en phase gazeuse H₂S, NH₃ et H₂O. L'indène hydrogéné totalement reste dans l'effluent.

Un gas-oil atmosphérique auquel a été ajouté 2,41 % en poids de composé polysulfure organique de type TPS37® a été utilisé comme charge de sulfuration. Les conditions opératoires de l'étape de sulfuration sont identiques à celles du test catalytique lui-même tel que cela est décrit ci-dessus.

### Exemple 1 : démarrage non conforme C1

La température du catalyseur est montée de la température ambiante (20°C) jusqu'à 150°C sous hydrogène pur. Ensuite, la charge de sulfuration (c'est-à-dire le gas-oil additionné d'un composé polysulfure organique de type TPS37®) est injectée à un débit de 80 cc/h. La température du catalyseur est alors augmentée jusqu'à 250°C à la vitesse de 10 °C/min puis un palier est respecté jusqu'à ce que la quantité de composé polysulfure organique de type TPS37® soit égale 0,54 kg par kg de soufre nécessaire à la sulfuration théorique complète du catalyseur. La température est ensuite augmentée jusqu'à 350°C à la vitesse de 10°C/min et un palier est respecté jusqu'à ce que la quantité de composé polysulfure organique de type TPS37® soit égale 3.24 kg par kg de soufre nécessaire la sulfuration théorique complète du catalyseur. Ensuite. la température est baissée jusqu'à la première température de test c'est-à-dire jusqu'à 310°C moment auquel la charge de sulfuration est remplacée par la charge à traiter c'est-à-dire par le napthalène de type carbochimique dont l'analyse est reportée dans le tableau 1.

La montée en température dans cet exemple effectué en pilote est réalisée à une vitesse beaucoup plus rapide que pour les réalisation à l'échelle industrielle.

### Exemple 2 : démarrage phase liquide conforme C2

La température du catalyseur est montée de la température ambiante (20°C) jusqu'à 150°C sous hydrogène pur. Ensuite, la charge de sulfuration (c'est-à-dire le gas-oil additionné du composé polysulfure organique de type TPS37®) est injectée à un débit de 80cc/h. La température du catalyseur est alors augmentée jusqu'à 250°C à la vitesse de 10°C/min puis un palier est respecté jusqu'à ce que la quantité de composé polysulfure organique de type TPS37® soit égale 1,62 kg par kg de soufre nécessaire à la sulfuration théorique complète du catalyseur (au lieu de 0,54 kg seulement dans l'exemple 1). La température est ensuite augmentée jusqu'à 350°C à la vitesse de 10°C/min et un palier est respecté jusqu'à ce que la quantité de composé polysulfure organique de type TPS37® soit égale à 3,24 kg par kg de soufre nécessaire la sulfuration théorique complète. Ensuite, la température est baissée jusqu'à la première température de test c'est-à-dire jusqu'à 310°C moment auquel la charge de sulfuration est remplacée par la charge à traiter c'est-à-dire par le naphtalène de type carbochimique dont l'analyse est reportée dans le tableau 1.

La montée en température dans cet exemple effectué en pilote est réalisée à une vitesse beaucoup plus rapide que pour les réalisation à l'échelle industrielle.

Dans le tableau 2, nous avons reporté l'analyse de l'effluent ainsi obtenu après hydrotraitement, strippage, distillation et enfin cristallisation pour les deux types de démarrage.

**Tableau 2**

| | non conforme C1 | conforme C2 |
|---|---|---|
| Soufre (ppm poids) | 23 | 10 |
| Naphtalène (%pds) | > 99% | > 99% |
| Tétraline (%pds) | 0,025 | 0.025 |
| Décaline (%poids) | < 0,001 | < 0,001 |
| Phénols (%poids) | 0,0011 | < 0,001 |
| Indène (%poids) | < 0,001 | < 0,001 |
| 1&2 méthyl-naphtalène (%poids) | 0,005 | 0,005 |
| Quinoleine (%poids) | 0,10 | 0,07 |
| Point de fusion (°C) | 78 | > 80,2 |

Comme on peut le voir, l'utilisation d'un démarrage conforme à notre invention permet d'atteindre un degré d'activité HDS supérieure puisque la teneur en soufre finale est de 10 ppm de soufre contre 23 dans le cas d'un démarrage non conforme, une hydrodéazotation HDN supérieure (0,07 % poids de quinoléïne lorsqu'on utilise le catalyseur C2 contre 0,10% poids de quinoléïne lorsqu'on utilise le catalyseur C1), une hydrodéhydroxylation supérieure (<0,001% poids de phénols lorsqu'on utilise le catalyseur C2 contre 0,0011 % poids de phénols lorsqu'on utilise le catalyseur C1). Enfin, l'amélioration du point de fusion permet de constater de façon globale une meilleure qualité de produit.

Nous avons aussi étudié les performances catalytiques c'est-à-dire la stabilité et l'activité de ces 2 catalyseurs.

Les performances catalytiques sont regroupées dans le tableau 3. Pour chaque démarrage (avec les catalyseurs C1 et C2), nous avons mesuré l'activité et la stabilité. L'activité est ici illustrée par la température de réacteur qu'il faut pour obtenir un naphtalène ayant un point de fusion au moins égal à 80,2 °C et la stabilité est exprimée en mois de fonctionnement du catalyseur sans qu'une perte d'efficacité soit à noter.

**tableau 3 :**

| | Exemple 1 (non conforme) | Exemple 2 (conforme) |
|---|---|---|
| Temps pour naphtalène pur | 332°C | 318 °C |
| durée de cycle (mois) | 15 | 34 |

## Revendications

1. Procédé de sulfuration d'un catalyseur contenant au moins de l'alumine et au moins un métal du groupe VIII à l'état d'oxydes caractérisé en ce qu'il consiste à injecter sur le catalyseur au moins un polysulfure organique choisi dans le groupe formé par les polysulfures organiques de type TPS 32®, TPS 37®, TPS 40®, TPS 54®, de façon telle que la quantité, en masse, de polysulfure organique injectée avant que la température du catalyseur atteigne 250°C, par rapport à la quantité. en masse, de soufre nécessaire à la sulfuration complète dudit catalyseur soit au moins égale à 1,56 pour les polysulfures organiques de type TPS 32®, au moins égale à 1,35 pour les polysulfures organiques de type TPS 37®, au moins égale à 1,25 pour les polysulfures organiques de type TPS 40®, et au moins égale à 0,93 pour les polysulfures organiques de type TPS 54®.

2. Procédé selon la revendication 1 caractérisé en ce que ledit catalyseur contient au moins un métal du groupe VIB à l'état d'oxydes.

3. Procédé selon la revendication 1 ou 2 caractérisé en ce que la concentration totale en oxydes de métaux est comprise entre 5 et 40 % en poids.

4. Procédé selon l'une des revendications précédentes caractérisé en ce que la matrice du catalyseur utilisé à l'étape d'hydrotraitement comprend au moins un élément choisi dans le groupe constitué par la silice-alumine, l'oxyde de bore, la magnésie, la zircone, les argiles et leurs mélanges.

5. Procédé selon l'une des revendications précédentes caractérisé en ce que le catalyseur comprend du phosphore.

6. Procédé selon l'une des revendications précédentes caractérisé en ce que le catalyseur utilisé à l'étape d'hydrotraitement présente les caractéristiques suivantes : sa surface spécifique BET est d'au plus 220 m²/g, son volume poreux est compris entre 0,35 et 0,7 ml/g, le diamètre moyen de ses pores est d'au moins 100Å.

7. Procédé selon l'une des revendications précédentes caractérisé en ce qu'il comprend un traitement à l'hydrogène du catalyseur, effectuée avant, pendant ou après la sulfuration.

8. Procédé de sulfuration selon l'une des revendications précédentes caractérisé en ce que l'étape de sulfuration est précédée de l'étape suivante : le catalyseur est soumis à une atmosphère d'hydrogène à température comprise entre la température ambiante et une température inférieure à 250°C, le débit d'hydrogène étant compris entre 1 et 10000 litres de gaz par litre de catalyseur.

9. Catalyseur obtenu par le procédé de sulfuration selon l'une des revendications 1 à 8.

10. Procédé de purification des coupes naphtalèniques comprenant au moins une étape d'hydrotraitement effectuée en présence d'un catalyseur sulfuré suivant le procédé de sulfuration selon l'une des revendications 1 à 8.

11. Procédé de purification selon la revendication 10 caractérisé en ce qu'il comprend ensuite successivement au moins une étape dans laquelle une partie au moins de l'effluent qui a subi l'hydrotraitement est débarrassé au moins en partie sinon en totalité des molécules de H₂S, NH₃ et H₂O générées, au moins une étape pour récupérer la plus grande quantité de naphtalène avec la pureté la plus élevée possible.
